# EUROPÄISCHE PATENTSCHRIFT

(11) **EP 2 550 003 B1**
(45) Veröffentlichungstag und Bekanntmachung des Hinweises auf die Patenterteilung: **21.12.2016**
(21) Anmeldenummer: 11709884.8
(22) Anmeldetag: 18.03.2011
(51) Int. Cl.: A61K 31/724, A61P 7/08

(54) **PHARMAZEUTISCHE ZUSAMMENSETZUNGEN ENTHALTEND SUBSTITUIERTES 6-DEOXY-6-SULFANYLCYCLODEXTRIN**
COMPOSITIONS COMPRISING SUBSTITUTED 6-DEOXY-6-SULFANYLCYCLODEXTRINS
COMPOSITIONS COMPRENANT DES 6-DEOXY-6-SULFANYLCYCLODEXTRINES SUBSTITUÉES

(30) Priorität: 22.03.2010 DE 102010012281
(43) Veröffentlichungstag der Anmeldung: 30.01.2013
(73) Patentinhaber: Fresenius Medical Care Deutschland GmbH, 61352 Bad Homburg (DE)
(72) Erfinder: FICHERT, Thomas, 48231 Warendorf (DE); GERBER, Johannes, 55127Mainz (DE); BICHLMAIER, Ingo, 61206 Wöllstadt (DE)
(74) Vertreter: Breuninger, Marcus
(86) Internationale Anmeldenummer: PCT/EP2011/001356
(87) Internationale Veröffentlichungsnummer: WO 2011/116909

(56) Entgegenhaltungen:
- WO-A1-01/40316
- WO-A2-02/36105
- WO-A2-2005/042584
- WO-A2-2006/001844
- BAER H H ET AL: "Heptakis[6-S-(2,3-dihydroxypropyl)-6-thio ] cyclomaltoheptaose and its sulfone: water-soluble beta-cyclodextrin derivatives having modified polarity", CARBOHYDRATE RESEARCH, PERGAMON, GB, Bd. 280, Nr. 2, 11. Januar 1996 (1996-01-11), Seiten 315-321, XP004018822, ISSN: 0008-6215, DOI: DOI:10.1016/0008-6215(95)00310-X

## Beschreibung

Die vorliegende Erfindung betrifft pharmazeutische Zusammensetzungen enthaltend substituierte 6-Deoxy-6-sulfanylcyclodextrine sowie deren Verwendung als Osmotika, insbesondere zur Verwendung in der Dialysebehandlung.

Osmotisch aktive Verbindungen (Osmotika) finden in der Pharmazie und Medizin breite Anwendung. So werden Osmotika beispielsweise zur Einstellung der Tonizität von Arzneimitteln, insbesondere parenteralen Medikamenten verwendet. Hierbei wird der osmotische Druck eines Arzneimittels je nach Art der Anwendung hypotonisch, hypertonisch oder isotonisch eingestellt. Beispielsweise kann der osmotische Druck einer parenteralen Arzneilösung dem osmotischen Druck des menschlichen Bluts durch Zugabe eines Osmotikums angeglichen werden (isoosmotische Lösungen).

Ferner werden Osmotika bei der Dialysebehandlung, insbesondere bei der Peritonealdialyse eingesetzt, um dem Dialysepatienten überschüssiges Wasser zu entziehen.

Das Peritonealdialyseverfahren beruht darauf, dass eine Lösung, die osmotisch aktive Verbindungen enthält, über einen Katheter in die Bauchhöhle des Dialysepatienten eingebracht wird. Diese Lösung wird für eine bestimmte Zeit (üblicherweise einige Stunden) in der Bauchhöhle des Patienten belassen und entfaltet dort ihre osmotische Wirkung; d.h. dem Patienten wird körpereigenes Wasser in die Bauchhöhle entzogen. Nach einer bestimmten Verweildauer wird die nunmehr verdünnte Peritonealdialyselösung über einen Katheter abgelassen.

Dieses Prinzip findet in verschiedenen Verfahren der Peritonealdialysebehandlung Anwendung. Nach Bedarf können beispielsweise die Verfahren der intermittierenden (IPD), nächtlichen intermittierenden (NIPD), kontinuierlichen zyklischen (CCPD) oder kontinuierlichen ambulanten Peritonealdialyse (CAPD), angewendet werden. Bei IPD, NIPD und CCPD kommen Geräte zum Einsatz, die den Patienten bei der Durchführung des Peritonealdialyseverfahrens unterstützen. Die CAPD ist ein manuelles Verfahren.

Durch die Zugabe von osmotisch aktiven Verbindungen soll insbesondere gewährleistet werden, dass der osmotische Druck der Peritonealdialyselösung während der gesamten Verweildauer in der Bauchhöhle hoch genug ist, um dem Patienten Wasser zu entziehen; d.h. Wasser geht aus dem Kreislauf des Patienten in die Bauchhöhle über (Ultrafiltration).

Jedoch kommt es aufgrund des Wasserübertritts in die Bauchhöhle zwangsläufig zu einer Verdünnung der eingebrachten Peritonealdialyselösung. Diese Verdünnung hat zur Folge, dass die Konzentration der osmotisch aktiven Verbindung und somit auch der osmotische Druck dieser Lösung abnehmen.

Nimmt der osmotische Druck der Peritonealdialyselösung aufgrund dieser Verdünnung ab, so hat dies wiederum zur Folge, dass auch der pro Zeiteinheit stattfindende Wasserübertritt in die Bauchhöhle abnimmt oder möglicherweise gänzlich zum Erliegen kommt. In diesen Fällen findet also mit fortschreitender Verweildauer der Peritonealdialyselösung in der Bauchhöhle des Patienten kein effektiver Wasserentzug mehr statt.

Durch Absorption von osmotisch aktiven Verbindungen in den Blutkreislauf des Patienten, kann sich die Richtung des Wasserübertritts sogar umkehren, d.h. Wasser geht aus der Bauchhöhle in den Blutkreislauf des Patienten über (negative Ultrafiltration). Dies ist dann der Fall, wenn die verdünnte Peritonealdialyselösung in der Bauchhöhle einen geringeren osmotischen Druck als das körpereigene Wasser (z.B. das Blut) des Patienten aufweist.

Durch Zugabe geeigneter osmotisch aktiver Verbindungen zur Peritonealdialyselösung kann der osmotische Druck über eine für die Peritonealdialyse geeignete Behandlungsdauer aufrechterhalten werden, so dass es innerhalb der Verweildauer der Lösung in der Bauchhöhle zu keinem übermäßigen Rückgang der Ultrafiltration kommt. Somit wird auch eine negative Ultrafiltration weitestgehend verhindert.

Die in der Peritonealdialysebehandlung eingesetzten Lösungen enthalten in der Regel Zuckermonomere oder -polymere, wie beispielsweise Glucose oder Polyglucose (z.B. Stärkederivate), als osmotisch wirksame Verbindungen.

US4889634 betrifft eine Peritonealdialyselösung, die Hydroxypropyl-β-cyclodextrin enthält.

JP8071146 betrifft eine Peritonealdialyselösung, die α- oder γ-Cyclodextrin, 2-Hydroxy-ethylether-, 2-Hydroxypropylether-, 6-*O*-α-Glucosyl- oder 6-O-α-Maltosylderivate von α-, β-und y-Cyclodextrin enthält.

Der Stand der Technik beschreibt jedoch Cyclodextrinderivate, die nichtselektiv substituiert sind. Vielmehr handelt es sich bei diesen Cyclodextrinderivaten um Gemische verschiedenster Verbindungen (unterschiedliche Substitutionsgrade, unterschiedliche Stellungsisomere), da die Substitution der Cyclodextrine nur bedingt selektiv an bestimmten Hydroxygruppen der Cyclodextrine erfolgt.

Eine Aufgabe der vorliegenden Erfindung ist es, Cyclodextrinderivate für pharmazeutische Zusammensetzungen zur Verfügung zu stellen, die gegenüber den Cyclodextrinderivaten des Standes der Technik ein einheitlicheres Substitutionsmuster aufweisen; d.h. dass beispielsweise das Vorhandensein unterschiedlicher Stellungsisomere erniedrigt bzw. ausgeschlossen wird.

Eine weitere Aufgabe der vorliegenden Erfindung ist es, Cyclodextrinderivate zur Verfügung zu stellen, die eine hohe Wasserlöslichkeit, eine verbesserte osmotische Wirksamkeit und eine erhöhte Ultrafiltration gegenüber den Cyclodextrinen des Standes der Technik aufweisen, und die somit für pharmazeutische Zusammensetzungen insbesondere zur Dialysebehandlung geeignet sind.

Diese Aufgabe wird durch den Gegenstand der Patentansprüche gelöst.

Die erfindungsgemäßen 6-Deoxy-6-sulfanylcyclodextrine zeichnen sich gegenüber den Cyclodextrinderivaten des Standes der Technik durch ein einheitlicheres Substitutionsmuster aus, d.h. sie sind in ihrer Struktur genauer definiert (z.B. geringerer Anteil unterschiedlicher Stellungsisomere; einheitlicher Substitutionsgrad).

Dies hat zur Folge, dass unerwünschte Nebenwirkungen, die auf dem Vorhandensein verschiedenster unterschiedlicher Substitutionsmuster beruhen, weitestgehend ausgeschlossen werden. Dies erhöht die Sicherheit für den Patienten.

Insbesondere kann auch die Wirksamkeit der erfindungsgemäßen 6-Deoxy-6-sulfanylcyclodextrine auf definierte Verbindungen zurückgeführt werden und beruht nicht auf den Wirkungen eines komplexen Gemisches unterschiedlichster Cyclodextrinderivate. Auch dies erhöht die Sicherheit für den Patienten und erleichtert insbesondere die Evaluierung pharmakologischer bzw. klinischer Daten.

Zudem können Gemische an substituierten Cyclodextrinderivaten, die nur eine eingeschränkt reproduzierbare Zusammensetzung aufweisen, zu mangelnder Reproduzierbarkeit bei der Ermittlung experimenteller Daten führen, wie beispielsweise bei der Durchführung pharmakologischer oder toxikologischer *in vivo-* oder *ex* vivo-Experimenten sowie bei der Durchführung klinischer Studien.

Ein erster Gegenstand dieser Erfindung betrifft 6-Deoxy-6-sulfanylcyclodextrine der allgemeinen Struktur I 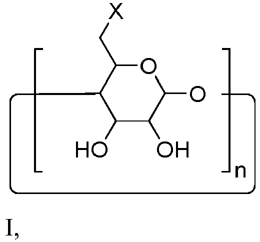 wobei
a) X für -S-R oder -OH steht
b) n für 6, 7 oder 8 steht,
c) der Substitutitonsgrad bezogen auf den Rest -S-R ≥0,0001 und ≤n ist und
d) R ausgewählt ist aus der Gruppe -C₁₋₆-Alkyl-NR¹R²,
   - C₁₋₆-Alkyl-N⁺R³R⁴R⁵, -C₁₋₆-Alkyl-COOH, -C(=O)-C₁₋₆-Alkyl-COOH, -C(=O)-CH₂-C(OH)(COOH)-CH₂-COOH, dihydroxyliertes C₁₋₆-Alkyl, C₁₋₆-Alkyl-PO₃H₂ und
   - C₁₋₆-Alkyl-SO₃H, wobei R¹, R², R³, R⁴ und R⁵, unabhängig voneinander, für -H oder -C₁₋₃-Alkyl stehen,
zur Verwendung als Osmotikum.

Ein bevorzugter Gegenstand dieser Erfindung betrifft 6-Deoxy-6-sulfanylcyclodextrine der allgemeinen Struktur II 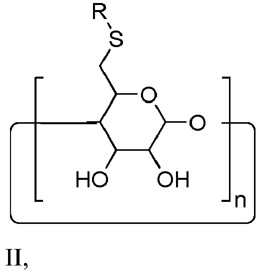 wobei
a) n für 6, 7 oder 8 steht und
b) R ausgewählt ist aus der Gruppe -C₁₋₆-Alkyl-NR¹R²,
   - C₁₋₆-Alkyl-N⁺R³R⁴R⁵, -C₁₋₆-Alkyl-COOH, -C(=O)-C₁₋₆-Alkyl-COOH, -C(=O)-CH₂-C(OH)(COOH)-CH₂-COOH, dihydroxyliertes C₁₋₆-Alkyl, C₁₋₆-Alkyl-PO₃H₂ und
   - C₁₋₆-Alkyl-SO₃H, wobei R¹, R², R³, R⁴ und R⁵, unabhängig voneinander, für -H oder -C₁₋₃-Alkyl stehen,
zur Verwendung als Osmotikum.

Der Ausdruck "C₁₋₆-Alkyl" umfasst im Sinne dieser Erfindung acyclische, gesättigte oder ungesättigte Kohlenwasserstoffreste, die verzweigt- oder geradkettig sein können und 1, 2, 3, 4, 5 oder 6 Kohlenstoffatome enthalten. Vorzugsweise umfasst C₁₋₆-Alkyl die Reste C₁₋₆-Alkanyl, C₂₋₆-Alkenyl und C₂₋₆-Alkinyl. Dabei weisen C₂₋₆-Alkenyle mindestens eine C-C-Doppelbindung und C₂₋₆-Alkinyle mindestens eine C-C-Dreifachbindung auf, wohingegen C₁₋₆-Alkanyle vollständig gesättigt sind. Vorzugsweise ist C₁₋₆-Alkyl ausgewählt aus der Gruppe, die Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, iso-Butenyl, n-Butenyl, cis-2-Butenyl, trans-2-Butenyl, 1,2-Butadienyl, 1,3-Butadienyl, Pent-1-enyl, cis-Pent-2-enyl, trans-Pent-2-enyl, 2-Methyl-but-1-enyl, 2-Methyl-but-2-enyl, 3-Methyl-but-1-enyl, Hex-1-enyl, Hex-2-enyl und Hex-3-enyl enthält.

Der Ausdruck "C₁₋₃-Alkyl" umfasst im Sinne dieser Erfindung acyclische, gesättigte oder ungesättigte Kohlenwasserstoffreste, die verzweigt- oder geradkettig sein können und 1, 2 oder 3 Kohlenstoffatome enthalten. Vorzugsweise umfasst C₁₋₃-Alkyl die Reste C₁₋₃-Alkanyl, C₂₋₃-Alkenyl und C₂₋₃-Alkinyl. Dabei weisen C₂₋₃-Alkenyle mindestens eine C-C-Doppelbindung und C₂₋₃-Alkinyle mindestens eine C-C-Dreifachbindung auf, wohingegen C₁₋₃-Alkanyle vollständig gesättigt sind. Vorzugsweise ist C₁₋₃-Alkyl ausgewählt aus der Gruppe, die Methyl, Ethyl, iso-Propyl, n-Propyl, Ethenyl, Ethinyl und Prop-1-enyl enthält.

Der basische Rest -C₁₋₆-Alkyl-NR¹R² kann protoniert, d.h. kationisch vorliegen und mit Anionen - beispielsweise Fluorid, Chlorid, Bromid, Iodid, Hydrogencarbonat, Carbonat, Dihydrogenphosphat, Hydrogenphosphat, Phosphat, Lactat, Pyruvat - als Salz vorliegen.

Die Gruppe -NR¹R² kann an jede mögliche Stelle des -C₁₋₆-Alkyls gebunden sein.

In einer bevorzugten Ausführungsform ist der Rest -C₁₋₆-Alkyl-NR¹R² ausgewählt aus der Gruppe -C₁₋₆-Alkyl-N(Methyl)₂, -C₁₋₆-Alkyl-N(Ethyl)₂, -C₁₋₆-Alkyl-N(Propyl)₂ und -C₁₋₆-Alkyl-N(Isopropyl)₂.

In einer weiteren bevorzugten Ausführungsform ist der Rest -C₁₋₆-Alkyl-NR¹R² ausgewählt aus der Gruppe -CH₂-N(Methyl)₂, -CH₂-N(Ethyl)₂, -CH₂-N(Propyl)₂, -CH₂-N(Isopropyl)₂, -(CH₂)₂-N(Methyl)₂, -(CH₂)₂-N(Ethyl)₂, -(CH₂)₂-N(Propyl)₂, -(CH₂)₂-N(Isopropyl)₂, -(CH₂)₃-N(Methyl)₂, -(CH₂)₃-N(Ethyl)₂, -(CH₂)₃-N(Propyl)₂, -(CH₂)₃-N(Isopropyl)₂, -(CH₂)₄-N(Methyl)₂, -(CH₂)₄-N(Ethyl)₂, -(CH₂)₄-N(Propyl)₂, -(CH₂)₄-N(Isopropyl)₂, -(CH₂)₅-N(Methyl)₂, -(CH₂)₅-N(Ethyl)₂, -(CH₂)₅-N(Propyl)₂, -(CH₂)₅-N(Isopropyl)₂, -(CH₂)₆-N(Methyl)₂, -(CH₂)₄-N(Ethyl)₆, -(CH₂)₄-N(Propyl)₆ und -(CH₂)₆-N(Isopropyl)₂.

Der Rest -C₁₋₆-Alkyl-N⁺R³R⁴R⁵ kann mit Anionen - beispielsweise Fluorid, Chlorid, Bromid, Iodid, Hydrogencarbonat, Carbonat, Dihydrogenphosphat, Hydrogenphosphat, Phosphat, Lactat, Pyruvat - als Salz vorliegen.

Die Gruppe -N⁺R³R⁴R⁵ kann an jede mögliche Stelle des -C₁₋₆-Alkyls gebunden sein.

Der Rest -C₁₋₆-Alkyl-N⁺R³R⁴R⁵ ist vorzugsweise ausgewählt aus der Gruppe -CH₂-Alkyl-N⁺(Methyl)₃, -CH₂-Alkyl-N⁺(Ethyl)₃, -CH₂-Alkyl-N⁺(Propyl)₃, -CH₂-Alkyl-N⁺(Isopropyl)₃, -(CH₂)₂-Alkyl-N⁺(Methyl)₃, -(CH₂)₂-Alkyl-N⁺(Ethyl)₃, -(CH₂)₂-Alkyl-N⁺(Propyl)₃, -(CH₂)₂-Alkyl-N⁺(Isopropyl)₃, -(CH₂)₃-Alkyl-N⁺(Methyl)₃, -(CH₂)₃-Alkyl-N⁺(Ethyl)₃, -(CH₂)₃-Alkyl-N⁺(Propyl)₃, -(CH₂)₃-Alkyl-N⁺(Isopropyl)₃, -(CH₂)₄-Alkyl-N⁺(Methyl)₃, -(CH₂)₄-Alkyl-N⁺(Ethyl)₃, -(CH₂)₄-Alkyl-N⁺(Propyl)₃, -(CH₂)₄-Alkyl-N⁺(Isopropyl)₃, -(CH₂)₅-Alkyl-N⁺(Methyl)₃, -(CH₂)₅-Alkyl-N⁺(Ethyl)₃, -(CH₂)₅-Alkyl-N⁺(Propyl)₃ und -(CH₂)₅-Alkyl-N⁺(Isopropyl)₃.

Die sauren Reste -C₁₋₆-Alkyl-COOH, -C(=O)-C₁₋₆-Alkyl-COOH, -C(=O)-CH₂-C(OH)(COOH)-CH₂-COOH (Zitronensäurerest), C₁₋₆-Alkyl-PO₃H₂ und -C₁₋₆-Alkyl-SO₃H können deprotoniert, d.h. anionisch vorliegen und mit Kationen - beispielsweise Natrium, Kalium, Magnesium, Calcium, Ammonium - als Salz vorliegen.

Die Reste -COOH, -PO₃H₂ und SO₃H können an jede mögliche Stelle des -C₁₋₆-Alkyls gebunden sein.

In einer bevorzugten Ausführungsform ist der Rest -C₁₋₆-Alkyl-COOH ausgewählt aus der Gruppe -CH₂-COOH, -(CH₂)₂-COOH, -(CH₂)₃-COOH, -(CH₂)₄-COOH, -(CH₂)₅-COOH und -(CH₂)₆-COOH.

Der Rest -C(=O)-C₁₋₆-Alkyl-COOH ist vorzugsweise ausgewählt aus der Gruppe -C(=O)-CH₂-COOH, -C(=O)-(CH₂)₂-COOH, -C(=O)-(CH₂)₃-COOH, -C(=O)-(CH₂)₄-COOH, -C(=O)-(CH₂)₅-COOH und -C(=O)-(CH₂)₆-COH.

In einer bevorzugten Ausführungsform ist der Rest -C₁₋₆-Alkyl-PO₃H₂ ausgewählt aus der Gruppe -CH₂-PO₃H₂, -(CH₂)₂-PO₃H₂, -(CH₂)₃-PO₃H₂, -(CH₂)₄-PO₃H₂, -(CH₂)₅-PO₃H₂, -(CH₂)₆-PO₃H₂, -CH=CH-PO₃H₂, -CH₂-CH=CH-PO₃H₂, -(CH₂)₂-CH=CH-PO₃H₂, -(CH₂)₃-CH=CH-PO₃H₂ und -(CH₂)₄-CH=CH-PO₃H₂.

Der Rest -C₁₋₆-Alkyl-SO₃H ist vorzugsweise ausgewählt aus der Gruppe -CH₂-SO₃H, -(CH₂)₂-SO₃H, -(CH₂)₃-SO₃H, -(CH₂)₄-SO₃H, -(CH₂)₅-SO₃H und -(CH₂)₆-SO₃H.

Im Sinne dieser Beschreibung steht der Begriff "Substitutionsgrad" für die durchschnittliche Molzahl von Rest R bezogen auf 1 Mol Cyclodextrin.

In einer bevorzugten Ausführungsform ist der Substitutionsgrad des erfindungsgemäßen 6-Deoxy-6-sulfanylcyclodextrins ≥1 und ≤n.

In einer weiteren bevorzugten Ausführungsform ist der Substitutionsgrad des erfindungsgemäßen 6-Deoxy-6-sulfanylcyclodextrins ≥n-1 und ≤n.

Für den Fall, dass n für 6 steht, kann der Substitutionsgrad Werte von >0 bis höchstens 6 (dies entspricht 6 Substituenten pro 6-Deoxy-6-sulfanyl-α-cyclodextrin-Molekül) annehmen.

Für den Fall, dass n für 7 steht, kann der Substitutionsgrad Werte >0 bis höchstens 7 (dies entspricht 7 Substituenten pro 6-Deoxy-6-sulfanyl-β-cyclodextrin-Molekül) annehmen.

Für den Fall, dass n für 8 steht, kann der Substitutionsgrad Werte von >0 bis höchstens 8 (dies entspricht 8 Substituenten pro 6-Deoxy-6-sulfanylcyclodextrin-Molekül) annehmen.

Im Fall, dass n = 6 ist, hat der Substitutionsgrad vorzugsweise einen Wert von 1 bis 6 oder 2 bis 6, bevorzugter 3 bis 6, noch bevorzugter 4 bis 6, am Bevorzugtesten 5 bis 6 oder 5,5 bis 6 und insbesondere 6.

Im Fall, dass n = 7 ist, hat der Substitutionsgrad vorzugsweise einen Wert von 1 bis 7 oder 2 bis 7, bevorzugter 3 bis 6 oder 4 bis 7, noch bevorzugter 5 bis 7, am Bevorzugtesten 6 bis 7 oder 6,5 bis 7 und insbesondere 7.

Im Fall, dass n = 8 ist, hat der Substitutionsgrad vorzugsweise einen Wert von 1 bis 8 oder 2 bis 8, bevorzugter 3 bis 8 oder 4 bis 8, noch bevorzugter 5 bis 8 oder 6 bis 8, am Bevorzugtesten 7 bis 8 oder 7,5 bis 8 und insbesondere 8.

Vorzugsweise weist eine 7,5 gewichtsprozentige wässrige Lösung des erfindungsgemäßen 6-Deoxy-6-sulfanylcyclodextrins eine theoretische Osmolarität ≥5 mosm/L, bevorzugter größer als ≥7,5 mosm/L, noch bevorzugter größer als ≥10 mosm/L, am Bevorzugtesten größer als ≥12,5 mosm/L und insbesondere größer als ≥15 mosm/L auf.

Zum Zwecke dieser Beschreibung steht der Ausdruck "theoretische Osmolarität" für die theoretisch berechnete Osmolarität. Methoden zur Berechnung dieses Werts sind dem Fachmann bekannt.

In einer bevorzugten Ausführungsform beträgt der kolloidosmotische Druck einer 7,5 gewichtsprozentigen Lösung des erfindungsgemäßen 6-Deoxy-6-sulfanylcyclodextrins ≥50 mosm/L oder ≥60 mosm/L, bevorzugter ≥70 mosm/L oder ≥80 mosm/L, noch bevorzugter ≥90 mosm/L oder ≥100 mosm/L, am Bevorzugtesten ≥110 mosm/L oder ≥120 mosm/L und insbesondere ≥130 mosm/L oder ≥140 mosm/L.

In einer weiteren bevorzugten Ausführungsform beträgt der kolloidosmotische Druck einer 7,5 gewichtsprozentigen Lösung des erfindungsgemäßen 6-Deoxy-6-sulfanylcyclodextrin ≥150 mosm/L oder ≥160 mosm/L, bevorzugter ≥170 mosm/L oder ≥180 mosm/L, noch bevorzugter ≥190 mosm/L oder ≥200 mosm/L, am Bevorzugtesten ≥210 mosm/L oder ≥220 mosm/L und insbesondere ≥230 mosm/L oder ≥240 mosm/L.

In einer weiteren bevorzugten Ausführungsform beträgt der kolloidosmotische Druck einer 7,5 gewichtsprozentigen Lösung des erfindungsgemäßen 6-Deoxy-6-sulfanylcyclodextrins 50 bis 500 mosm/L, bevorzugter 75 mosm/L bis 400 mosm/L, noch bevorzugter 100 bis 300 mosm/L, am Bevorzugtesten 110 mosm/L bis 275 mosm/L und insbesondere 120 mosm/L bis 250 mosm/L.

In einer weiteren bevorzugten Ausführungsform beträgt der kolloidosmotische Druck einer 7,5 gewichtsprozentigen Lösung des erfindungsgemäßen 6-Deoxy-6-sulfanylcyclodextrins 100 bis 500 mosm/L, bevorzugter 100 mosm/L bis 400 mosm/L, noch bevorzugter 100 bis 350 mosm/L, am Bevorzugtesten 100 mosm/L bis 325 mosm/L und insbesondere 100 mosm/L bis 290 mosm/L.

Zum Zwecke dieser Beschreibung steht der Ausdruck "kolloidosmotischer Druck" für den experimentell gemessenen osmotischen Druck der Lösung, der sich aus dem osmotischen und onkotischen Druck zusammensetzt. Geeignete Methoden zur experimentellen Bestimmung dieses Werts sind dem Fachmann bekannt.

Die Osmolalität einer 7,5 gewichtsprozentigen wässrigen Lösung des erfindungsgemäßen 6-Deoxy-6-sulfanylcyclodextrins beträgt vorzugsweise >5 mosm/kg, bevorzugter ≥7,5 mosm/kg, noch bevorzugter ≥10 mosm/kg, am Bevorzugtesten ≥12 mosm/kg und insbesondere ≥15 mosm/kg.

Zum Zwecke dieser Beschreibung steht der Begriff "Osmolalität" für die mittels Gefrierpunktserniedrigung experimentell bestimmten Osmolalität der Lösung. Methoden zur Bestimmung der Gefrierpunktserniedrigung sind dem Fachmann bekannt.

Die mittels Gefrierpunkterniedrigung experimentell bestimmte Osmolarität einer 7,5 gewichtsprozentigen wässrigen Lösung des erfindungsgemäßen 6-Deoxy-6-sulfanylcyclodextrins beträgt vorzugsweise ≥15 mosm/L, bevorzugter ≥17 mosm/L, noch bevorzugter ≥19 mosm/L, am Bevorzugtesten ≥21 mosm/L und insbesondere ≥23 mosm/L.

Das erfindungsgemäße 6-Deoxy-6-sulfanylcyclodextrin eignet sich vorzugsweise als Osmotikum zur Einstellung der Tonizität von Arzneimitteln, insbesondere von Arzneilösungen zur parenteralen Applikation.

In einer bevorzugten Ausführungsform findet das erfindungsgemäße 6-Deoxy-6-sulfanylcyclodextrin Verwendung in der Dialysebehandlung, vorzugsweise in der Hämo- und/oder Peritonealdialysebehandlung.

Das erfindungsgemäße 6-Deoxy-6-sulfanylcyclodextrin ist insbesondere zur Verwendung in der Peritonealdialysebehandlung geeignet.

Ein weiterer Gegenstand dieser Erfindung betrifft Dialyselösungen enthaltend wenigstens ein erfindungsgemäßes 6-Deoxy-6-sulfanylcyclodextrin.

In einer bevorzugten Ausführungsform ist die erfindungsgemäße Dialyselösung eine Hämodialyselösung oder eine Peritonealdialyselösung. Die erfindungsgemäße Dialyselösung ist insbesondere eine Peritonealdialyselösung.

Darreichungsformen, die in der Dialysebehandlung eingesetzt werden, sind vorzugsweise Konzentrate in Mehrkomponenten-Systemen oder gebrauchsfertige Dialyselösungen.

Für die Zwecke dieser Erfindung umfasst der Ausdruck "Dialyselösung" eine gebrauchsfertige Darreichungsform zur Dialysebehandlung, d.h. eine flüssige Zubereitung, die als solche zur Applikation geeignet ist. Insbesondere muss die Dialyselösung vor der Applikation nicht verdünnt und/ oder mit anderen Zubereitungen gemischt werden.

Im Gegensatz zu den vorstehend beschriebenen Dialyselösungen werden Konzentrate, die entweder in flüssiger, halbfester oder fester Form vorliegen können, vor der Applikation mit Wasser oder wässrigen Lösungen verdünnt oder in Wasser oder wässrigen Lösungen gelöst. In analoger Weise müssen die Komponenten eines Mehrkomponenten-Systems vor der Applikation miteinander gemischt werden um eine gebrauchsfertige Dialyselösung zu erhalten. Konzentrate und Mehrkomponenten-Systeme können also als Vorstufe der erfindungsgemäßen Dialyselösung angesehen werden.

Die erfindungsgemäße Dialyselösung ist vorzugsweise eine Hämodialyse- oder eine Peritonealdialyselösung. Hämodialyse- und Peritonealdialyselösungen enthalten üblicherweise Elektrolyte in einer Konzentration, die im Wesentlichen der Plasma-Elektrolyt-Konzentration entspricht. Elektrolyte umfassen üblicherweise Natrium-, Kalium-, Calcium-, Magnesium- und Chlorid-Ionen.

Dialyselösungen haben üblicherweise einen physiologisch verträglichen pH-Wert. Dies wird vorzugsweise erreicht durch Puffer (Puffer-Systeme), die selbst zum Gesamtgehalt an Elektrolyten beitragen können. Die Puffer sind vorzugsweise Hydrogencarbonat, Lactat oder Pyruvat.

Ferner besitzen Dialyselösungen üblicherweise eine physiologisch verträgliche Osmolarität. Dies wird in der Regel erreicht durch die in der Dialyselösung enthaltenen Elektrolyte und erfindungsgemäßen 6-Deoxy-6-sulfanylcyclodextrine, die als osmotisch aktive Verbindungen (Osmotika) in der gewünschten Konzentration physiologisch verträglich sind.

Die erfindungsgemäße Dialyselösung besitzt eine Osmolarität im Bereich von vorzugsweise 200 bis 550 mosm/L.

Im Fall, dass die erfindungsgemäße Dialyselösung eine Hämodialyselösung ist, beträgt die Osmolarität vorzugsweise 200 bis 350 mosm/L oder 210 bis 340 mosm/L, bevorzugter 220 bis 330 mosm/L, noch bevorzugter 230 bis 320 mosm/L, am Bevorzugtesten 240 bis 310 mosm/L und insbesondere 250 bis 300 mosm/L. Verfahren zur Messung der Osmolarität und des osmotischen Drucks sind dem Fachmann bekannt. Beispielsweise können diese mit Hilfe eines Membranosmometers oder anderen geeigneten Messmethoden bestimmt werden.

Im Fall, dass die erfindungsgemäße Dialyselösung eine Peritonealdialyselösung ist, beträgt die Osmolarität vorzugsweise 200 bis 570 mosm/L oder 210 bis 560 mosm/L, bevorzugter 220 bis 550 mosm/L, noch bevorzugter 230 bis 540 mosm/L, am Bevorzugtesten 240 bis 530 mosm/L und insbesondere 250 bis 520 mosm/L. In einer bevorzugten Ausführungsform beträgt die Osmolarität 250 ± 50 mosm/L oder 250 ± 45 mosm/L, bevorzugter 250 ± 35 mosm/L, noch bevorzugter 250 ± 25 mosm/L, am Bevorzugtesten 250 ± 15 mosm/L und insbesondere 250 ± 10 mosm/L. In einer weiteren bevorzugten Ausführungsform beträgt die Osmolarität 300 ± 50 mosm/L oder 300 ± 45 mosm/L, bevorzugter 300 ± 35 mosm/L, noch bevorzugter 300 ± 25 mosm/L, am Bevorzugtesten 300 ± 15 mosm/L und insbesondere 300 ± 10 mosm/L. In einer weiteren bevorzugten Ausführungsform beträgt die Osmolarität 350 ± 50 mosm/L oder 350 ± 45 mosm/L, bevorzugter 350 ± 35 mosm/L, noch bevorzugter 350 -± 25 mosm/L, am Bevorzugtesten 350 ± 15 mosm/L und insbesondere 300 ± 10 mosm/L. In einer weiteren bevorzugten Ausführungsform beträgt die Osmolarität 400 ± 50 mosm/L oder 400 ± 45 mosm/L, bevorzugter 400 ± 35 mosm/L, noch bevorzugter 400 ± 25 mosm/L, am Bevorzugtesten 400 ± 15 mosm/L und insbesondere 300 ± 10 mosm/L. In einer weiteren bevorzugten Ausführungsform beträgt die Osmolarität 450 ± 50 mosm/L oder 450 ± 45 mosm/L, bevorzugter 450 ± 35 mosm/L, noch bevorzugter 450 ± 25 mosm/L, am Bevorzugtesten 450 ± 15 mosm/L und insbesondere 450 ± 10 mosm/L. In einer weiteren bevorzugten Ausführungsform beträgt die Osmolarität 500 ± 50 mosm/L oder 500 ± 45 mosm/L, bevorzugter 500 ± 35 mosm/L, noch bevorzugter 500 ± 25 mosm/L, am Bevorzugtesten 500 ± 15 mosm/L und insbesondere 500 ± 10 mosm/L.

Die erfindungsgemäße Dialyselösung hat einen pH-Wert vorzugsweise von 4,0 bis 8,0, bevorzugter von 4,2 bis 7,5, noch bevorzugter von 4,4 bis 6,8, am Bevorzugtesten von 4,6 bis 6,0 oder 4,8 bis 5,5 und insbesondere von 5,0 bis 5,2 oder 5,0±0,1; gemessen bei Raumtemperatur (20 bis 23 °C). In einer bevorzugten Ausführungsform ist der pH-Wert 4,8 ± 1,0 oder 4,8 ± 0.8, bevorzugter 4,8 ± 0,7 oder 4,8 ± 0,6, noch bevorzugter 4,8 ± 0,5 oder 4,8 ± 0,4, am Bevorzugtesten 4,8 ± 0,3 oder 4,8 ± 0,2 und insbesondere 4,8 ± 0,1. In einer weiteren bevorzugten Ausführungsform ist der pH-Wert 5,0 ± 1,0 oder 5,0 ± 0.8, bevorzugter 5,0 ± 0,7 oder 5,0 ± 0,6, noch bevorzugter 5,0 ± 0,5 oder 5,0 ± 0,4, am Bevorzugtesten 5,0 ± 0,3 oder 5,0 ± 0,2 und insbesondere 5,0 ± 0,1. In einer weiteren bevorzugten Ausführungsform ist der pH-Wert 5,2 ± 1,0 oder 5,2 ± 0,8, bevorzugter 5,2 ± 0,7 oder 5,2 ± 0,6, noch bevorzugter 5,2 ± 0,5 oder 5,2 ± 0,4, am Bevorzugtesten 5,2 ± 0,3 oder 5,2 ± 0,2 und insbesondere 5,2 ± 0,1. In einer weiteren bevorzugten Ausführungsform ist der pH-Wert 5,5 ± 1,0 oder 5,5 ± 0,8, bevorzugter 5,5 ± 0,7 oder 5,5 ± 0,6, noch bevorzugter 5,5 ± 0,5 oder 5,5 ± 0,4, am Bevorzugtesten 5,5 ± 0,3 oder 5,5 ± 0,2 und insbesondere 5,5 ± 0,1. In einer weiteren bevorzugten Ausführungsform ist der pH-Wert 6,0 ± 1,0 oder 6,0 ± 0.8, bevorzugter 6,0 ± 0,7 oder 6,0 ± 0,6, noch bevorzugter 6,0 ± 0,5 oder 6,0 ± 0,4, am Bevorzugtesten 6,0 ± 0,3 oder 6,0 ± 0,2 und insbesondere 6,0 ± 0,1. In einer weiteren bevorzugten Ausführungsform ist der pH-Wert 6,5 ± 1,0 oder 6,5 ± 0.8, bevorzugter 6,5 ± 0,7 oder 6,5 ± 0,6, noch bevorzugter 6,5 ± 0,5 oder 6,5 ± 0,4, am Bevorzugtesten 6,5 ± 0,3 oder 6,5 ± 0,2 und insbesondere 6,5 ± 0,1. In einer weiteren bevorzugten Ausführungsform ist der pH-Wert 7,0 ± 1,0 oder 7,0 ± 0.8, bevorzugter 7,0 ± 0,7 oder 7,0 ± 0,6, noch bevorzugter 7,0 ± 0,5 oder 7,0 ± 0,4, am Bevorzugtesten 7,0 ± 0,3 oder 7,0 ± 0,2 und insbesondere 7,0 ± 0,1. In einer weiteren bevorzugten Ausführungsform ist der pH-Wert 7,4 ± 1,0 oder 7,4 ± 0.8, bevorzugter 7,4 ± 0,7 oder 7,4 ± 0,6, noch bevorzugter 7,4 ± 0,5 oder 7,4 ± 0,4, am Bevorzugtesten 7,4 ± 0,3 oder 7,4 ± 0,2 und insbesondere 7,4 ± 0,1. In einer weiteren bevorzugten Ausführungsform ist der pH-Wert 8,0 ± 1,0 oder 8,0 ± 0.8, bevorzugter 8,0 ± 0,7 oder 8,0 ± 0,6, noch bevorzugter 8,0 ± 0,5 oder 8,0 ± 0,4, am Bevorzugtesten 8,0 ± 0,3 oder 8,0 ± 0,2 und insbesondere 8,0 ± 0,1.

Die erfindungsgemäße Dialyselösung enthält ein oder mehrere (z.B. zwei, drei, vier oder fünf) erfindungsgemäße 6-Deoxy-6-sulfanylcyclodextrine; wobei die erfindungsgemäßen 6-Deoxy-6-sulfanylcyclodextrine wie oben stehend definiert sind.

Die erfindungsgemäße Dialyselösung enthält erfindungsgemäßes 6-Deoxy-6-sulfanyl-cyclodextrin in einer Gesamtkonzentration von vorzugsweise 0,001 mM bis 10 M oder 0,01 bis 1,0 M, bevorzugter 0,10 bis 500 mM, noch bevorzugter 1,0 bis 250 mM, am Bevorzugtesten 10 bis 100 mM und insbesondere 25 bis 90 mM. In einer bevorzugten Ausführungsform ist die Gesamtkonzentration 25 ± 24 mM, bevorzugter 25 ± 20 mM, noch bevorzugter 25 ± 15 mM, am Bevorzugtesten 25 ± 10 mM und insbesondere 25 ± 5 mM. In einer weiteren bevorzugten Ausführungsform ist die Gesamtkonzentration 50 ± 25 mM, bevorzugter 50 ± 20 mM, noch bevorzugter 50 ± 15 mM, am Bevorzugtesten 50 ± 10 mM und insbesondere 50 ± 5 mM. In einer weiteren bevorzugten Ausführungsform ist die Gesamtkonzentration 75 ± 25 mM, bevorzugter 75 ± 20 mM, noch bevorzugter 75 ± 15 mM, am Bevorzugtesten 75 ± 10 mM und insbesondere 75 ± 5 mM. In einer weiteren bevorzugten Ausführungsform ist die Gesamtkonzentration 100 ± 25 mM, bevorzugter 100 ± 20 mM, noch bevorzugter 100 ± 15 mM, am Bevorzugtesten 100 ± 10 mM und insbesondere 100 ± 5 mM. In einer weiteren bevorzugten Ausführungsform ist die Gesamtkonzentration 200 ± 25 mM, bevorzugter 200 ± 20 mM, noch bevorzugter 200 ± 15 mM, am Bevorzugtesten 200 ± 10 mM und insbesondere 200 ± 5 mM. Die Gesamtkonzentration ist vorzugsweise berechnet mittels des mittleren Molekulargewichts der erfindungsgemäßen 6-Deoxy-6-sulfanylcyclodextrine.

Die erfindungsgemäße Dialyselösung enthält erfindungsgemäßes 6-Deoxy-6-sulfanylcyclodextrin in einer Gesamtmassenkonzentration von vorzugsweise 0,01 g/L bis 1,0 kg/L, bevorzugter von 0,1 bis 750 g/L, noch bevorzugter von 1,0 bis 500 g/L, am Bevorzugtesten 10 bis 250 g/L und insbesondere von 100 bis 200 g/L. In einer bevorzugten Ausführungsform ist die Gesamtmassenkonzentration 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

Die erfindungsgemäße Dialyselösung kann auch weitere osmotisch aktive Substanzen wie beispielsweise Glucose, Polyglucose, quervernetzte Glucose oder Polyglucose, Mannitol oder Glycerol enthalten.

Die erfindungsgemäße Dialyselösung enthält vorzugsweise einen oder mehrere Elektrolyte.

Im Sinne dieser Erfindung steht der Ausdruck "Elektrolyt" für eine Substanz, die freie Ionen enthält und elektrische Konduktivität aufweist. Vorzugsweise dissoziiert das Elektrolyt vollständig in Kationen und Anionen ohne den pH-Wert einer wässrigen Zusammensetzung im Wesentlichen zu ändern. Diese Eigenschaft grenzt Elektrolyte von Puffersubstanzen ab. Vorzugsweise liegen die Elektrolyte in einer Konzentration vor, die in einer im Wesentlichen vollständigen Dissoziation in Wasser resultiert.

Bevorzugte Elektrolyte sind ausgewählt aus der Gruppe der Alkalimetalle wie beispielsweise Na⁺ und K⁺ und der Erdalkalimetalle wie beispielsweise Ca²⁺ und Mg²⁺. Ein bevorzugtes Anion ist Cl⁻.

Die erfindungsgemäße Dialyselösung kann weitere Anionen wie beispielsweise Hydrogencarbonat, Dihydrogenphosphat, Hydrogenphosphat, Phosphat, Acetat, Lactat und Pyruvat enthalten; diese Anionen (in geeigneten Kombinationen mit Kationen) werden jedoch aufgrund Ihrer Pufferkapazität im Sinne dieser Erfindung nicht als Elektrolyte sondern als Puffer bezeichnet.

In einer bevorzugten Ausführungsform enthält die erfindungsgemäße Dialyselösung Na⁺-Ionen. Die Konzentration an Na⁺-Ionen ist vorzugsweise 10 bis 200 mM oder 50 bis 190 mM, bevorzugter 100 bis 180 mM oder 110 bis 170 mM, noch bevorzugter 115 bis 165 mM oder 120 bis 160 mM, am Bevorzugtesten 125 bis 155 mM und insbesondere 130 bis 150 mM. In einer weiteren bevorzugten Ausführungsform enthält die erfindungsgemäße Dialyselösung keine Na⁺-Ionen.

In einer bevorzugten Ausführungsform enthält die erfindungsgemäße Dialyselösung K⁺-Ionen. Die Konzentration an K⁺-Ionen ist vorzugsweise 0,10 bis 20 mM, bevorzugter 0,25 bis 15 mM, noch bevorzugter 0,50 bis 10 mM, am Bevorzugtesten 0,75 bis 7,5 mM und insbesondere 1,0 bis 5,0 mM. In einer weiteren bevorzugten Ausführungsform ist die Konzentration an K⁺-Ionen 1,0 ± 0,75, 2,0 ± 0,75, 3,0 ± 0,75, 4,0 ± 0,75 oder 5,0 ± 0,75 mM und insbesondere 1,0 ± 0,50, 2,0 ± 0,50, 3,0 ± 0,50, 4,0 ± 0,50 oder 5,0 ± 0,50. In einer weiteren bevorzugten Ausführungsform enthält die erfindungsgemäße Dialyselösung keine K⁺-Ionen.

In einer bevorzugten Ausführungsform enthält die erfindungsgemäße Dialyselösung Ca²⁺-Ionen. Die Konzentration an Ca²⁺-Ionen ist vorzugsweise 0,1 bis 3 mM, bevorzugter 0,25 bis 2,75 mM, noch bevorzugter 0,5 bis 2,5 mM, am Bevorzugtesten 0,75 bis 2,25 mM und insbesondere 1 bis 2 mM. In einer weiteren bevorzugten Ausführungsform ist die Konzentration an Ca²⁺-Ionen 0,25, 0,5, 0,75, 1, 1,25, 1,5, 1,75 oder 2 mM. In einer weiteren bevorzugten Ausführungsform enthält die erfindungsgemäße Dialyselösung keine Ca²⁺-Ionen.

In einer bevorzugten Ausführungsform enthält die erfindungsgemäße Dialyselösung Mg²⁺-Ionen. Die Konzentration an Mg²⁺-Ionen ist vorzugsweise 0,01 bis 1 mM, bevorzugter 0,05 bis 0,75 mM, noch bevorzugter 0,1 bis 0,5 mM, am Bevorzugtesten 0,15 bis 0,4 mM und insbesondere 0,2 bis 0,3 mM. In einer weiteren bevorzugten Ausführungsform ist die Konzentration an Mg²⁺-Ionen 0,05, 0,075, 0,1, 0,2, 0,25, 0,50 oder 0,75 mM. In einer weiteren bevorzugten Ausführungsform enthält die erfindungsgemäße Dialyselösung keine Mg²⁺-Ionen.
In einer bevorzugten Ausführungsform enthält die erfindungsgemäße Dialyselösung Cl⁻-Ionen. Die Konzentration an Cl⁻-Ionen ist vorzugsweise 10 bis 300 mM, bevorzugter 25 bis 250 mM, noch bevorzugter 50 bis 200 mM, am Bevorzugtesten 75 bis 150 mM und insbesondere 80 bis 125 mM. In einer weiteren bevorzugten Ausführungsform ist die Konzentration an Cl⁻-Ionen 100 ± 50 mM, bevorzugter 100 ± 25 mM, am Bevorzugtesten 100 ± 10 mM und insbesondere 96 ± 4 mM. In einer weiteren bevorzugten Ausführungsform enthält die erfindungsgemäße Dialyselösung keine Cl⁻-Ionen.

Die erfindungsgemäße Dialyselösung enthält vorzugsweise einen oder mehrere Puffer.

Geeignete Puffer sind dem Fachmann bekannt. Üblicherweise umfassen Puffer Lactat-, Hydrogencarbonat-, Carbonat-, Dihydrogenphosphat-, Hydrogenphosphat-, Phosphat-, Pyruvat-, Citrat-, Isocitrat-, Succinat-, Fumarat-, Acetat- und Lactat-Salze. Der Fachmann weiß, dass das entsprechende Kation der vorstehend genannten Anionen Bestandteil des Puffers ist, der zur Einstellung des pH-Wertes benutzt wird (z.B. Na^{⊕} als Bestandteil des Puffers NaHCO₃). Wenn das Puffersalz jedoch in Wasser dissoziiert hat es auch die Wirkung eines Elektrolyts. Für die Zwecke dieser Beschreibung berechnen sich die Konzentrationen an Kationen oder Anionen und die Gesamtkonzentration an Ionen, unabhängig davon, ob sie als Bestandteil von Elektrolyten, Puffern oder anderen Verbindungen (z.B. als Salz der erfindungsgemäßen 6-Deoxy-6-sulfanylcyclodextrine) eingesetzt werden.

In einer bevorzugten Ausführungsform enthält der Puffer Hydrogencarbonat. Hydrogencarbonat ist ein gut verträgliches Puffersystem, das im alkalischen Milieu mit Carbonat und im sauren Milieu mit H₂CO₃ bzw. CO₂ im Gleichgewicht steht. Neben Hydrogencarbonat sind auch andere Puffer-Systeme einsetzbar, die im Bereich von pH 4 bis pH 8, bevorzugter im Bereich von pH 5 bis pH 7,6 und insbesondere im Bereich von pH 7,6, 7,4, 7,2 und/oder 7,0 eine Pufferwirkung ausüben; z.B. auch Verbindungen, die im Körper zu Hydrogencarbonat metabolisiert werden können wie Lactat oder Pyruvat.

In einer weiteren bevorzugten Ausführungsform enthält der Puffer das Salz einer schwachen Säure, vorzugsweise Lactat. Die Säurestärke (pKₛ) der schwachen Säure ist vorzugsweise ≤5. Der Puffer kann auch ein Gemisch von Substanzen mit Pufferwirkung sein, z.B. ein Gemisch enthaltend Hydrogencarbonat und ein Salz einer schwachen Säure (z.B. Lactat). Eine geringe Hydrogencarbonat-Konzentration hat den Vorteil, dass der CO₂-Druck im Behältnis gering ist.

In einer bevorzugten Ausführungsform ist die erfindungsgemäße Dialyselösung gepuffert durch Hydrogencarbonat. Die Hydrogencarbonat-Konzentration ist vorzugsweise 1,0 bis 200 mM, bevorzugter 2,5 bis 150 mM, noch bevorzugter 5 bis 100 mM, am Bevorzugtesten 5 bis 75 mM oder 10 bis 50 mM und insbesondere 20 bis 30 mM. In einer weiteren bevorzugten Ausführungsform ist die Hydrogencarbonat-Konzentration 25 mM. In einer weiteren bevorzugten Ausführungsform enthält die erfindungsgemäße Dialyselösung kein Hydrogencarbonat.

In einer bevorzugten Ausführungsform ist die erfindungsgemäße Dialyselösung gepuffert durch Lactat. Die Lactat-Konzentration ist vorzugsweise 1,0 bis 200 mM, bevorzugter 2,5 bis 150 mM, noch bevorzugter 5 bis 100 mM, am Bevorzugtesten 10 bis 50 mM oder 10 bis 25 mM und insbesondere 15 mM. In einer weiteren bevorzugten Ausführungsform enthält die erfindungsgemäße Dialyselösung kein Lactat.

In einer bevorzugten Ausführungsform ist die erfindungsgemäße Dialyselösung gepuffert durch Acetat. Die Acetat-Konzentration ist vorzugsweise 1,0 bis 100 mM, bevorzugter 1,0 bis 50 mM, noch bevorzugter 1,0 bis 25 mM, am Bevorzugtesten 1,0 bis 10 mM oder 2,0 bis 7,5 mM und insbesondere 2,5 bis 7,0 mM. In einer weiteren bevorzugten Ausführungsform enthält die erfindungsgemäße Dialyselösung kein Acetat.

Das Gesamtvolumen an Dialyselösung ist nicht beschränkt. Üblicherweise beträgt das Volumen mehrere Liter (geeignetes Verabreichungsvolumen für einen Patienten) bis einige hundert Liter (geeignetes Vorratsvolumen für mehr als einen Patienten).

Wie bereits oben stehend ausgeführt ist unter dem Ausdruck "Dialyselösung" im Sinne dieser Erfindung eine gebrauchsfertige Dialyselösung zu verstehen, d.h. die Dialyselösung kann direkt für die Dialysebehandlung (Hämodialyse oder Peritonealdialyse) verwendet werden.

In einer bevorzugten Ausführungsform ist die erfindungsgemäße Dialyselösung eine Peritonealdialyselösung wie nachstehend beschrieben.

Die Peritonealdialyselösung ist biochemisch so abgestimmt, dass sie die mit Nierenversagen einhergehende metabolische Azidose im Wesentlichen korrigiert. Die Peritonealdialyselösung enthält Hydrogencarbonat vorzugsweise in annähernd physiologischen Konzentrationen. In einer bevorzugten Ausführungsform enthält die Peritonealdialyselösung Hydrogencarbonat in einer Konzentration von ca. 20 bis 30 mM. In einer weiteren bevorzugten Ausführungsform enthält die Peritonealdialyselösung eine Hydrogencarbonat-Konzentration von 25 mM.

Ferner enthält die Peritonealdialyselösung vorzugsweise Kohlenstoffdioxid mit einem Partialdruck (pCO₂) von weniger als 60 mmHg. In einer bevorzugten Ausführungsform ist pCO₂ der Peritonealdialyselösung im Wesentlichen gleich zum pCO₂, der in Blutgefäßen gemessen wird.

Ferner hat die Peritonealdialyselösung vorzugsweise einen pH-Wert von ca. 7,4. Daher ist die Peritonealdialyselösung eine physiologisch verträgliche Lösung.

Die Peritonealdialyselösung enthält vorzugsweise eine schwache Säure mit einem pKₛ ≤5. Die schwachen Säuren sind vorzugsweise Verbindungen, die als physiologische Stoffwechselprodukte im Glucose-Metabolismus auftreten. Die schwache Säure ist vorzugsweise ausgewählt aus Gruppe bestehend aus Lactat, Pyruvat, Citrat, Isocitrat, Ketoglutarat, Succinat, Fumarat, Malat und Oxaloacetat. Diese Säuren können entweder allein oder als Gemisch in der Peritonealdialyselösung enthalten sein. Die schwachen Säuren sind vorzugsweise in einer Konzentration von 10 bis 20 mEq/L und im Wesentlichen als NatriumSalze in der Peritonealdialyselösung enthalten. In der Peritonealdialyselösung ist die schwache Säure vorzugsweise in einer Menge enthalten, die der täglichen metabolischen Wasserstoffproduktion von ca. 1 mEq/kg/Tag entspricht.

Die Peritonealdialyselösung enthält wenigstens ein erfindungsgemäßes 6-Deoxy-6-sulfanylcyclodextrin wie oben stehend definiert.

Die erfindungsgemäße Peritonealdialyselösung enthält vorzugsweise eine Konzentration an Hydrogencarbonat und weist einen pCO₂ auf, wie sie bei gesunden, nicht-niereninsuffizienten Patienten gemessen werden. Die schwache Säure diffundiert entlang des Konzentrationsgradienten von der Dialyselösung ins Blut des Dialysepatienten und korrigiert somit die metabolische Azidose der Dialysepatienten.

Ein weiterer Gegenstand dieser Erfindung betrifft Mehrkomponenten-Systeme zur Herstellung der oben beschriebenen gebrauchsfertigen Dialyselösungen. Die Herstellung erfolgt vorzugsweise in einer detailliert beschriebenen Art und Weise, d.h. durch Befolgung einer entsprechenden Anleitung (Protokoll). Besagte Herstellung kann manuell, z.B. durch Mischen einzelner Komponenten oder Verdünnen einer Komponente mit Wasser, erfolgen. Die Herstellung kann jedoch auch automatisiert erfolgen, z.B. mittels einer Vorrichtung die für diesen Prozess geeignet ist und kommerziell erhältlich sein kann. Die Herrichtung muss nicht zwangsläufig zu einer Dialyselösung mit statischer (gleich bleibender) Zusammensetzung führen sondern kann auch zu einer Dialyselösung führen, die kontinuierlich ihre Zusammensetzung ändert, wobei diese Änderung durch eine geeignete Vorrichtung überwacht werden kann. Beispielsweise kann das erfindungsgemäße 6-Deoxy-6-sulfanylcyclodextrin in einer Dialyselösung enthalten sein, die kontinuierlich während der Dialysebehandlung verdünnt wird, so dass der Patient einer abnehmenden 6-Deoxy-6-sulfanylcyclodextrin-Konzentration ausgesetzt ist.

In einer bevorzugten Ausführungsform sind die erfindungsgemäßen Dialyselösungen zur Verwendung in der Behandlung der Niereninsuffizienz geeignet.

In einer weiteren bevorzugten Ausführungsform sind die erfindungsgemäßen Dialyselösungen zur Verwendung in der Dialysebehandlung geeignet.
In einer weiteren bevorzugten Ausführungsform sind die erfindungsgemäßen Dialyselösungen zur Verwendung in der Hämodialyse- und / oder Peritonealdialysebehandlung geeignet.

Ein weiterer Gegenstand dieser Erfindung betrifft ein Kit, das für die Herstellung der erfindungsgemäßen Dialyselösungen konfiguriert ist, wobei das Kit
- eine erste Komponente,
- eine zweite Komponente und
- ggf. eine weitere oder mehrere weitere Komponenten
umfasst, und
die Herstellung der erfindungsgemäßen Dialyselösung durch Mischen der ersten Komponente mit der zweiten Komponente und ggf. der/ den weiteren Komponente(n) erfolgt.

Das Kit umfasst wenigstens eine erste Komponente und eine zweite Komponente. Das Kit kann auch weitere Komponenten umfassen, z.B. eine dritte und eine vierte Komponente. Vorzugsweise besteht das Kit aus zwei Komponenten, die vorzugsweise voneinander verschieden sind.

Im Sinne dieser Erfindung umfasst der Ausdruck "Komponente" flüssige, halbfeste oder feste Zusammensetzungen, die zueinander gleich oder voneinander verschieden sein können, wobei durch Mischen aller Komponenten des Kits die erfindungsgemäße gebrauchsfertige Dialyselösung erhalten wird. Vorzugsweise enthält eine einzelne Komponente einen Teil der Inhaltsstoffe, die in der gebrauchsfertigen Dialyselösung enthalten sind.

Die erste und die zweite Komponente können, unabhängig voneinander, fest, halbfest oder flüssig sein. Im Falle, dass die Komponenten flüssig sind, können sie Lösungen oder Dispersionen (z.B. Dispersionen oder Suspensionen) sein.

In einer bevorzugten Ausführungsform ist die erste Komponente flüssig, vorzugsweise reines Wasser oder eine wässrige Lösung, und die zweite Komponente ist ebenfalls flüssig. In einer weiteren bevorzugten Ausführungsform ist die erste Komponente flüssig, vorzugsweise reines Wasser oder eine wässrige Lösung, und die zweite Komponente ist fest, vorzugsweise ein pulverförmiges Gemisch.

Die erste Komponente ist vorzugsweise eine Lösung, die osmotisch wirksame Substanzen (z.B. erfindungsgemäßes 6-Deoxy-6-sulfanylcyclodextrin), Calcium-Ionen, Magnesium-Ionen, Hydronium-Ionen und Chlorid-Ionen enthält.

Das erfindungsgemäße Kit kann verschiedenartig ausgestaltet sein. Beispielsweise können die einzelnen Komponenten in voneinander separierten Behältnissen (z.B. einzelne Beutel) vorliegen. Das erfindungsgemäße Kit ist jedoch vorzugsweise ein Gebinde, wie beispielsweise ein Mehrkammer-Behältnis-System (z.B. flexibles oder starres Mehrkammer-Behältnis-System), vorzugsweise ein flexibles Mehrkammer-Beutel-System.

Das erfindungsgemäße Kit ist vorzugsweise ein Mehrkammer-Behältnis-System, das die erste Komponente, die zweite Komponente und ggf. eine oder mehrere weitere Komponenten in Kammern enthält, die voneinander durch lösbare bzw. brechbare Trennsysteme (z.B. Trennbrechteile) getrennt sind, wobei die erste Komponente, die zweite Komponente und ggf. die eine oder mehreren weiteren Komponenten nach dem Lösen bzw. Brechen des Trennsystems miteinander gemischt werden können, um die erfindungsgemäße Dialyselösung zu erhalten.

Das Mehrkammer-Behältnis kann als Kunststoffgebinde vorliegen (z.B. Mehrkammer-Kunststoffbeutel), der jeweils eine abgetrennte Kammer für jede einzelne Komponente enthält. Vorzugsweise enthält das Kunststoffgebinde die einzelnen Komponentenlösungen in Kammern, die jeweils durch Trennelemente voneinander abgetrennt sind.

Das Mehrkammer-Behältnis ist vorzugsweise ein Zweikammer-Beutel umfassend ein Kunststoffgebinde mit einer ersten Kammer und einer zweiten Kammer, wobei die Kammern durch ein lösbares bzw. brechbares Trennsystem voneinander getrennt sind, und die erste Kammer die erste Komponente enthält und die zweite Kammer die zweite Komponente enthält. Das Lösen bzw. Brechen des Trennsystems führt zum Mischen der beiden Komponenten und resultiert in der gebrauchsfertigen Dialyselösung. Die erste Kammer und die zweite Kammer sind im Gebinde vorzugsweise angrenzend angeordnet und durch das Trennsystem voneinander abgetrennt. Das Trennsystem ist vorzugsweise eine Trennnaht (z.B. lösbare oder brechbare Schweißnaht). Die Trennnaht öffnet sich vorzugsweise durch das Anlegen eines Druckes auf eine der Kammern, woraufhin die Trennnaht bricht bzw. sich löst und sich der Inhalt der beiden Kammern mischt und das Gemisch als gebrauchsfertige Dialyselösung in der Dialysebehandlung eingesetzt werden kann.

Die erste Komponente des erfindungsgemäßen Kits ist vorzugsweise eine sterile Lösung, die eine Säure enthält und einen pH-Wert ≤ 6,0 aufweist; die zweite Komponente ist vorzugsweise ebenfalls eine sterile Lösung, die vorzugsweise einen Puffer enthält und einen pH-Wert ≥ 7,0 aufweist.

Das erfindungsgemäße 6-Deoxy-6-sulfanylcyclodextrin kann in der ersten Komponente oder in der zweiten Komponente als auch in beiden Komponenten in gleichen oder verschiedenen Konzentrationen enthalten sein. In einer bevorzugten Ausführungsform ist das erfindungsgemäße 6-Deoxy-6-sulfanylcyclodextrin nur in der ersten (sauren) Komponente enthalten. In einer weiteren bevorzugten Ausführungsform ist das erfindungsgemäße 6-Deoxy-6-sulfanylcyclodextrin nur in der zweiten (basischen) Komponente enthalten. Die erste Komponente und/ oder die zweite Komponente und/ oder die ggf. weitere(n) Komponente(n) können einen oder mehrere Elektrolyte aber auch Puffer enthalten.

Der Fachmann erkennt, dass das Mischen der einzelnen Komponenten üblicherweise einen Verdünnungseffekt für den Fall nach sich zieht, dass die Komponenten die Inhaltsstoffe in unterschiedlichen Konzentrationen enthalten. Falls beispielsweise das erfindungsgemäße 6-Deoxy-6-sulfanylcyclodextrin ausschließlich in einer der Komponenten enthalten ist, führt das Mischen dieser Komponente mit wenigstens einer anderen Komponente zu einem Anstieg des Volumens in Bezug auf die vorhandene Menge des erfindungsgemäßen 6-Deoxy-6-sulfanylcyclodextrins und somit zu einer Verdünnung, d.h. Abnahme der 6-Deoxy-6-sulfanylcyclodextrin-Konzentration; folglich enthält die Komponente das erfindungsgemäße 6-Deoxy-6-sulfanylcyclodextrin vorzugsweise in einer höheren Konzentration als die gebrauchsfertige Dialyselösung.
Vorzugsweise ist die Konzentration an erfindungsgemäßen 6-Deoxy-6-sulfanylcyclodextrin in der Komponente nahe an der Sättigungskonzentration bei einer Temperatur von 5 °C um eine ausreichende Lagerstabilität bei höheren Temperaturen sicher zu stellen.

In einer bevorzugten Ausführungsform ist die Gesamtmassenkonzentration an erfindungsgemäßen 6-Deoxy-6-sulfanylcyclodextrin in der Komponente 0,01 g/L bis 1,0 kg/L, bevorzugter 0,1 bis 750 g/L, noch bevorzugter 1,0 bis 500 g/L, am Bevorzugtesten 10 bis 250 g/L und insbesondere 100 bis 200 g/L. In einer weiteren bevorzugten Ausführungsform ist die Gesamtmassenkonzentration an erfindungsgemäßen 6-Deoxy-6-sulfanylcyclodextrin in der Komponente 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration an erfindungsgemäßen 6-Deoxy-6-sulfanylcyclodextrin in der Komponente 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration an erfindungsgemäßen 6-Deoxy-6-sulfanylcyclodextrin in der Komponente 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration an erfindungsgemäßen 6-Deoxy-6-sulfanylcyclodextrin in der Komponente 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration an erfindungsgemäßen 6-Deoxy-6-sulfanylcyclodextrin in der Komponente 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

In einer bevorzugten Ausführungsform enthält die zweite Komponente die Gesamtmenge an erfindungsgemäßen 6-Deoxy-6-sulfanylcyclodextrin und einen geeigneten Puffer, der den pH-Wert der zweiten Komponente auf über 7,0, bevorzugter auf über 7,5, noch bevorzugter auf über 8,0, am Bevorzugtesten auf über 8,5 und insbesondere auf über 9,0 einstellt. Dies kann vorzugsweise durch Hydrogencarbonat erreicht werden, die beispielsweise in Form von dissoziiertem Natrium-Hydrogencarbonat und oder Kalium-Hydrogencarbonat vorliegen können. In einer weiteren bevorzugten Ausführungsform ist die zweite Komponente fest und umfasst ein pulverförmiges Gemisch enthaltend wenigstens ein erfindungsgemäßes 6-Deoxy-6-sulfanylcyclodextrin und wenigstens einen Puffer, z.B. Natrium- und/ oder Kalium-Hydrogencarbonat.

Der Mehrkammer-Beutel ist vorzugsweise geeignet für die Herstellung einer Dialyselösung, die zur Verwendung in der Peritonealdialysebehandlung verwendet werden kann, und die folgenden Inhaltsstoffe vorzugsweise in folgenden Konzentrationen enthält:

| | |
|---|---|
| Ca^{2⊕} | 0,5 bis 5 mval/L; |
| Mg^{2⊕} | 0 bis 3,0 mval/L; |
| Cl^{⊖} | 90,5 bis 121 mval/L; |
| K^{⊕} | 0 bis 4,0 mval/L; |
| HCO₃^{⊖} | 25 bis 40 mval/L; wobei |

eine Kammer des Mehrkammer-Beutel-Systems ein erstes saures Konzentrat und eine andere Kammer ein zweites basisches Konzentrat enthält; wobei das saure Konzentrat Ca2^{⊕}-Ionen enthält und das basische Konzentrat HCO₃^{⊖}-Ionen aber keine Ca^{2⊕}-Ionen enthält; und die zwei Konzentrate nach Lösen bzw. Brechen des Trennsystems (z.B. Trennnaht) miteinander gemischt werden können; wobei das Mischen der zwei Konzentrate zur Herstellung der gebrauchsfertigen Dialyselösung führt und der pH der gebrauchsfertigen Dialyselösung 7,0 bis 7,6 ist.

Vorzugsweise enthält das basische Konzentrat wenigstens ein erfindungsgemäßes 6-Deoxy-6-sulfanylcyclodextrin und ggf. Glucose und/ oder Polyglucose, wohingegen das saure Konzentrat kein erfindungsgemäßes 6-Deoxy-6-sulfanylcyclodextrin und keine Glucose und/ oder Polyglucose enthält.

Vorzugsweise enthält das basische Konzentrat eine Menge an Hydrogencarbonat, die zu einer Hydrogencarbonat-Konzentration der gebrauchsfertigen Dialyselösung von wenigstens 20 mM führt. Vorzugsweise ist die Hydrogencarbonat-Konzentration der basischen Komponente so hoch, dass die gebrauchsfertige Dialyselösung eine Hydrogencarbonat-Konzentration von 25 mM aufweist.

Der pH-Wert des basischen, gepufferten zweiten Konzentrats wird vorzugsweise mit Salzsäure eingestellt.

Vorzugsweise werden die beiden Konzentrate in einem Volumenverhältnis von 10:1 bis 1:10 oder 8:1 bis 1:8, bevorzugter 5:1 bis 1:5 oder 3:1 bis 1:3, noch bevorzugter 2:1 bis 1:2 und insbesondere 1:1 miteinander gemischt.

Der Mehrkammer-Beutel weist vorzugsweise eine Gasbarriere-Folie auf, die verhindert, das gasförmiges CO₂ aus dem System entweicht. Gasbarriere-Folien sind dem Fachmann bekannt.

Ein bevorzugter Gegenstand dieser Erfindung betrifft ein Verfahren zur Herstellung einer Dialyselösung, worin das gewünschte Mischverhältnis automatisch durch eine Dialysemaschine oder einen Peritonealdialyse-Cycler erfolgt.

In einer bevorzugten Ausführungsform betrifft die Erfindung eine feste Zusammensetzung, die zur Herstellung der erfindungsgemäßen Dialyselösung durch das Lösen in einem definierten Volumen eines Lösungsmittels (z.B. Wasser) geeignet ist. Vorzugsweise ist die feste Zusammensetzung eine vorstehend beschriebene Komponente und somit ein Bestandteil des erfindungsgemäßen Kits.

Die feste Zusammensetzung enthält das erfindungsgemäße 6-Deoxy-6-sulfanylcyclodextrin in einer beliebigen festen Form, z.B. als Pulver, Granulat, Pellets, etc. Das erfindungsgemäße 6-Deoxy-6-sulfanylcyclodextrin kann als Lyophilisat oder sprühgetrocknet vorliegen.

Die erfindungsgemäße feste Zusammensetzung enthält vorzugsweise ein Hydrogencarbonat-Salz wie beispielsweise Natrium- oder Kalium-Hydrogencarbonat. Das Stoffmengenverhältnis von Hydrogencarbonat zu erfindungsgemäßen 6-Deoxy-6-sulfanylcyclodextrin in der festen Zusammensetzung ist vorzugsweise 1:100 bis 100:1, bevorzugter 1:50 bis 50:1, noch bevorzugter 1:25 bis 25:1, am Bevorzugtesten 1:10 bis 10:1 und insbesondere 1:5 bis 5:1.

Das definierte Volumen an Lösungsmittel, das zur Herstellung der erfindungsgemäßen Dialyselösung durch Lösen der festen Zusammensetzung benötigt wird ist vorzugsweise 1,0 bis 2000 l. Vorzugsweise ist das Lösungsmittel gereinigtes Wasser, sterilisiertes Wasser oder Wasser für Injektionszwecke, das ggf. einen oder mehrere der oben beschriebenen Elektrolyte, eine oder mehrere osmotisch wirksame Substanzen (z.B. wenigstens ein erfindungsgemäßes 6-Deoxy-6-sulfanylcyclodextrin) und/ oder einen oder mehrere der oben beschriebenen Puffer enthalten kann.

Ein weiterer Gegenstand dieser Erfindung betrifft die Verwendung wenigstens eines erfindungsgemäßen 6-Deoxy-6-sulfanylcyclodextrins zur Herstellung der erfindungsgemäßen Dialyselösung (Hämodialyselösung oder Peritonealdialyselösung).

Ein weiterer Gegenstand dieser Erfindung betrifft die Verwendung eines erfindungsgemäßen Kits zur Herstellung der erfindungsgemäßen Dialyselösung (Hämodialyselösung oder Peritonealdialyselösung).

Ein weiterer Gegenstand dieser Erfindung betrifft die Verwendung einer erfindungsgemäßen festen Zusammensetzung zur Herstellung der erfindungsgemäßen Dialyselösung (Hämodialyselösung oder Peritonealdialyselösung).

### Beispiele

Die Verbindungen der Beispiele 1 bis 4 wurden gemäß einer publizierten Vorschrift hergestellt (Steffen et al., Chem. Eur. J. 2007, 13, 6801 - 6809).

Von jeder einzelnen Beispielverbindung wurde eine Charge mit niedrigem Substitutionsgrad von ca. 2 bis 3 und eine Charge mit hohem Substitutionsgrad von ca. 6 bis 7 hergestellt.

### Beispiel 1:

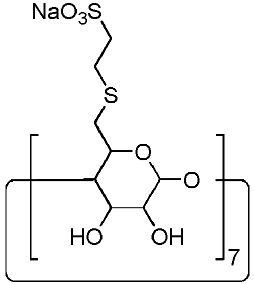

### Beispiel 2

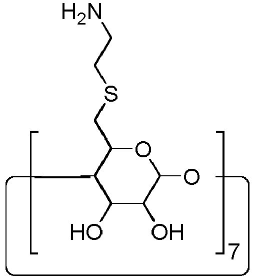

### Beispiel 3

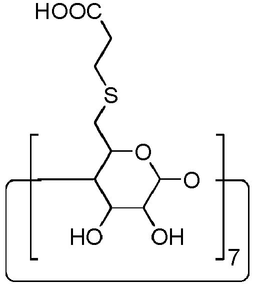

### Beispiel 4

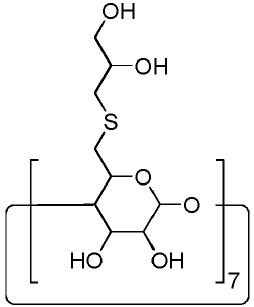

### Beispiel 5

In einem vergleichenden Versuch wurde ein Füllvolumen von 10 ml eines osmotischen Agens in einer Konzentration von 5 % (m/m) in einer Versuchslösung mit 1 mmol/l Ca2+, 0,5 mmol/l Mg2+, 138 mmol/l Na+, 106 mmol/l Cl- und 35 mmol/l Lactat in einen semipermeablen Schlauch (regenerierte Cellulose, MWCO: 1000, Fa. Roth) gefüllt und bei einer Temperatur von 38°C in einem Bad der gleichen Versuchslösung für 24 Stunden unter Bewegung gelagert. Zu verschiedenen Zeitpunkten wurde die Volumenzunahme des Füllvolumens des Schlauchs ermittelt, die die osmotische Wirkung des Agens widerspiegelt. Als osmotische Agenzien kamen ein Cyclodextrinderivat gemäß vorliegender Erfindung sowie die etablierten osmotischen Agenzien Glukose und Icodextrin zum Einsatz.
Das in dem Versuch verwendete Cyclodextrinderivat hat folgende Strukturformel: 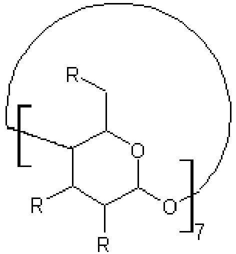 R = OH or S-CH₂-CH₂-COONa

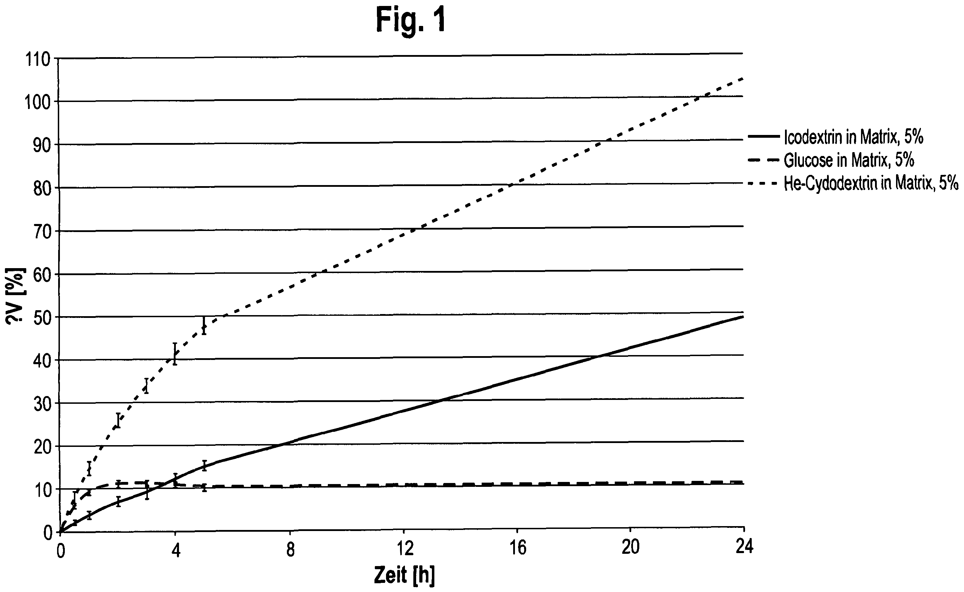

Die Ergebnisse sind in der Figur 1 als Diagramm dargestellt.
Das Cyclodextrinderivat zeigt gegenüber Glukose und Icodextrin eine stärkere osmotische Wirkung.

### Beispiel 6

Die Beispielverbindungen 1 bis 4 wurden mit den äquivalenten β-Cyclodextrinderivaten gemäß Beispiel 5 verglichen.

Die Beispielverbindungen zeigten eine geringere Anzahl an Stellungsisomeren und Verbindungen unterschiedlicher Molekulargewichte (analytisch beispielsweise mittels HPLC oder LC/MS/MS bestimmt) im Vergleich zu den äquivalenten Cyclodextrinverbindungen.

Gegenüber den Cyclodextrin-Derivaten der US4889634 und JP8071146 wiesen die Beispielverbindungen 1 bis 4 einen höheren kolloidosmotischen Druck und damit eine größere osmotische Wirksamkeit auf.

## Patentansprüche

1. 6-Deoxy-6-sulfanylcyclodextrin der allgemeinen Struktur I 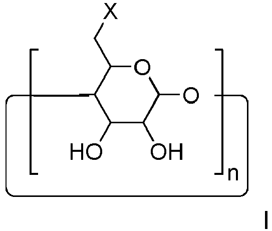 wobei
a) X für -S-R oder -OH steht,
b) n für 6, 7 oder 8 steht,
c) der Substitutitonsgrad bezogen auf den Rest -S-R ≥0,0001 und ≤n ist und
d) R ausgewählt ist aus der Gruppe -C₁₋₆-Alkyl-NR¹R²,
- C₁₋₆-Alkyl-N⁺R³R⁴R⁵, -C₁₋₆-Alkyl-COOH, -C(=O)-C₁₋₆-Alkyl-COOH, -C(=O)-CH₂-C(OH)(COOH)-CH₂-COOH, dihydroxyliertes C₁₋₆-Alkyl, C₁₋₆-Alkyl-PO₃H₂ und
- C₁₋₆-Alkyl-SO₃H, wobei R¹, R², R³, R⁴ und R⁵, unabhängig voneinander, für -H oder -C₁₋₃-Alkyl stehen,
zur Verwendung als Osmotikum.

2. 6-Deoxy-6-sulfanylcyclodextrin nach Anspruch 1, wobei der Substitutionsgrad ≥1 und ≤n ist.

3. 6-Deoxy-6-sulfanylcyclodextrin nach Anspruch 1 oder 2, wobei der Substitutionsgrad ≥n-1 und ≤n ist.

4. 6-Deoxy-6-sulfanylcyclodextrin nach einem oder mehreren der Ansprüche 1 bis 3, wobei eine 7,5 gewichtsprozentige wässrige Lösung des 6-Deoxy-6-sulfanylcyclodextrins eine Osmolarität ≥5 mosm/L aufweist.

5. 6-Deoxy-6-sulfanylcyclodextrin nach einem oder mehreren der Ansprüche 1 bis 4 zur Verwendung in der Dialysebehandlung .

6. 6-Deoxy-6-sulfanylcyclodextrin nach einem oder mehreren der Ansprüche 1 bis 4 zur Verwendung in der Hämodialyse- oder Peritonealdialysebehandlung.

7. Verwendung des 6-Deoxy-6-sulfanylcyclodextrins nach einem oder mehreren der Ansprüche 1 bis 4 zur Einstellung der Tonizität von Arzneimitteln.

## Claims

1. 6-Deoxy-6-sulfanylcyclodextrin of the general structure I 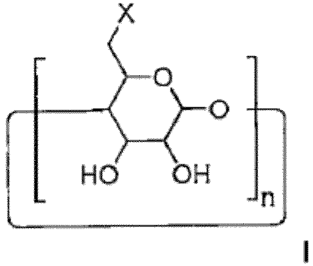 wherein
a) stands for -S-R or -OH,
b) n stands for 6, 7 or 8,
c) the degree of substitution, based on the -S-R radical, is ≥0.0001 and ≥n, and
d) R is selected from the groups -C₁₋₆-alkyl-NR¹R², -C₁₋₆-alkyl-N⁺R³R⁴R⁵, -C₁₋₆-alkyl-COOH,-C(=O)-C₁₋₈-alkyl-COOH, -C(=O)-CH₂-C(OH) (COOH)-CH₂-COOH, dihydroxylated C₁₋₆-alkyl, C₁₋₆-alkyl-PO₃H₂ and -C₁₋₆-alkyl-SO₃H, where R¹, R², R³, R⁴ and R⁵, independently of one another, stand for H or -C₁₋₃-alkyl,
for use as an osmotic agent.

2. The 6-deoxy-6-sulfanylcyclodextrin according to claim 1, wherein the degree of substitution is ≥1 and ≤n.

3. The 6-deoxy-6-sulfanylcyclodextrin according to claim 1 or 2, wherein the degree of substitution is ≥n-1 and ≤n.

4. The 6-deoxy-6-sulfanylcyclodextrin according to any one or more of claims 1 to 3, wherein a 7.5 % by weight aqueous solution of 6-deoxy-6-sulfanylcyclodextrin has an osmolarity of ≥5 mosm/L.

5. The 6-deoxy-6-sulfanylcyclodextrin according to any one or more of claims 1 to 4, for use in dialysis treatment.

6. The 6-deoxy-6-sulfanylcyclodextrin according to any one or more of claims 1 to 4, for use in hemodialysis or peritoneal dialysis treatment.

7. Use of 6-deoxy-6-sulfanylcyclodextrin according to any one or more of claims 1 to 4, for adjusting the tonicity of pharmaceutical drugs.

## Revendications

1. 6-déoxy-6-sulfanylcyclodextrine de structure générale 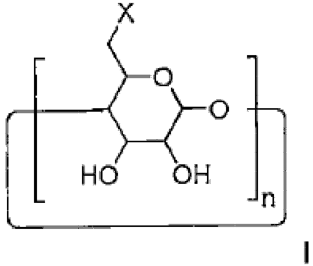 dans laquelle
a) X représente -S-R ou -OH,
b) n représente 6, 7 ou 8,
c) le degré de substitution par rapport au radical -S-R est >0,0001 et ≤ n et
d) R est sélectionné dans le groupe -C₁₋₆-alkyle-NR¹R², -C₁₋₆-alkyle N⁺R³R⁴R⁵, -C₁₋₆-alkyle-COOH, -C(=0)- C₁₋₆-alkyle-COOH, -C(=O)-CH₂-C(0H)(COOH)-CH₂-COOH, -C₁₋₆-alkyle dihydroxylé, -C₁₋₆-alkyle-PO₃H₂ et -C₁₋₆-alkyle-SO₃H, sachant que R¹, R², R³, R⁴ et R⁵, représentent, indépendamment les uns des autres, du -H ou-C₁₋₃-alkyle
à utiliser comme osmotique.

2. 6-déoxy-6-sulfanylcyclodextrine selon la revendication 1, dans laquelle le degré de substitution est ≥ 1 et ≤ n.

3. 6-déoxy-6-sulfanylcyclodextrine selon la revendication 1 ou 2, dans laquelle le degré de substitution est ≥ 1 et ≤ n.

4. 6-déoxy-6-sulfanylcyclodextrine selon une ou plusieurs des revendications 1 à 3, dans laquelle une solution aqueuse à 7,5 % en poids de 6-déoxy-6-sulfanylcyclodextrine présente une osmolarité de nous ≥ 5 mosm/L.

5. 6-déoxy-6-sulfanylcyclodextrine selon une ou plusieurs des revendications 1 à 4, destinée à une utilisation dans le traitement par dialyse.

6. 6-déoxy-6-sulfanylcyclodextrine selon une ou plusieurs des revendications 1 à 4, destinée à une utilisation dans le traitement par hémodialyse ou dialyse péritonéale.

7. Utilisation de la 6-déoxy-6-sulfanylcyclodextrine selon une ou plusieurs des revendications 1 à 4, destinée à l'ajustement de la tonicité des médicaments.
